(19) Europäisches Patentamt
European Patent Office
Office européen des brevets

(11) EP 1 438 971 A1

(12) EUROPEAN PATENT APPLICATION
published in accordance with Art. 158(3) EPC

(43) Date of publication:
21.07.2004 Bulletin 2004/30

(51) Int Cl.⁷: **A61K 45/00**, A61K 31/121,
A61K 33/26, A61P 37/04,
A61P 35/00

(21) Application number: 01980905.2

(22) Date of filing: 25.10.2001

(86) International application number:
PCT/JP2001/009381

(87) International publication number:
WO 2003/035108 (01.05.2003 Gazette 2003/18)

(84) Designated Contracting States:
AT BE CH CY DE DK ES FI FR GB GR IE IT LI LU
MC NL PT SE TR
Designated Extension States:
AL LT LV MK RO SI

(71) Applicant: TTC CO., Ltd.
Tokyo 150-0021 (JP)

(72) Inventors:
• KOBAYASHI, Takeshi
Nagoya-shi, Aichi 464-0096 (JP)

• SHINKAI, Masashige
Hatogaya-shi, Saitama 334-0013 (JP)
• HONDA, Hiroyuki
Nagoya-shi, Aichi 458-0818 (JP)
• UENO, K.; c/o Fuji Dormitory, No. 402
Nagoya-shi, Aichi 451-0077 (JP)
• OHTSUKA, Kenzo
Owariasahi-shi, Aichi 488-0825 (JP)

(74) Representative: Becker Kurig Straus
Patentanwälte
Bavariastrasse 7
80336 München (DE)

(54) **IMMUNOPOTENTIATORS IN THERMOTHERAPY FOR CANCER**

(57) The present invention relates to an immunostimulator in hyperthermia of cancer, which contains a heat shock protein-inducing compound such as geranylgeranyl acetone, etc. The immunostimulator of the present invention can effectively regress tumor tissue that is difficultly treated by hyperthermia alone by combining with the hyperthermia, and also, metastasis of cancer can be effectively inhibited substantially without side effect.

EP 1 438 971 A1

**Description**

(Technical field)

The present invention relates to hyperthermia of cancer, in particular, it relates to an immunostimulator in hyperthermia using magnetic fine particles. The immunostimulator of the present invention markedly improves therapeutic effects of cancer in hyperthermia.

(Background art)

At present, the main stream of cancer therapy is a surgical therapy. In the surgical therapy, the most significant problem is metastasis of cancer. Surgical operation is carried out by capturing tumor with naked eyes of a medical doctor oneself and it is removed. Thus, it cannot remove until the tumor becomes a certain size, but at such a stage, the cancer causes metastasis, or else, there is a possibility of not removing the tumor completely. Thus, it cannot help using an anticancer agent after the surgical operation. However, due to significant side effects, use of an anticancer agent is restricted in many cases.

As another therapeutic method of cancer, there is a hyperthermia. This is a therapeutic method of heating and killing tumor tissue by utilizing the characteristics that the tumor tissue has slightly high thermal sensitivity than a normal tissue at slightly higher temperature range (42°C to 45°C) than a body temperature. In the hyperthermia presently carried out, heating is carried out by irradiating a radio wave from outside the body which utilizes the slight difference in absorption of the ratio wave from that of a body tissue, so that there is a problem that a surface of the body is overheated. Thus, this is not a therapeutic method which can effectively treat a tumor at a deep portion or a small tumor alone.

The present inventors have already proposed a hyperthermia of cancer that uses magnetite with a order of submicrons as a heat generating material, as a novel therapeutic method of cancer in which the above-mentioned problems involved in the above-mentioned cancer therapy had been solved. This therapeutic method uses either magnetite cationic liposome (MCL) in which magnetite is coated with cationic phospholipid and optionally adhered by liposome that is fixed with an antibody specific to cancer cells or CMC magnetite in which magnetite is dispersed in a carboxymethylcellulose (CMC) solution, or uses needle-shaped molded magnetite that is molded and solidified in a needle-shape.

Magnetic fine particles such as MCL, etc. specifically adsorb to cancer cells. Thus, absorption of electromagnetic wave by tumor tissue is excellent than body tissue, and tumor tissue alone can be selectively heated, whereby hyperthermia due to inductive heat can be effectively carried out. According to this therapeutic method, it has been found that it has high antitumor effect by not only killing tumor tissue by heat, but also strengthening immune which is a prevention system in a body. In the immunostimulator, it has been considered to participate in heat shock protein (HSP).

However, even when the above-mentioned hyperthermia using magnetic fine particles such as MCL, etc. is employed, there are some cases in which therapeutic effects of cancer cannot be completely and sufficiently obtained.

Accordingly, in hyperthermia of cancer, it has been desired to obtain a manner which can markedly heighten therapeutic effects of cancer.

(Disclosure of the invention)

The present invention is based on the findings that in hyperthermia of cancer, a certain kind of a compound is administered to tumor tissue, induction of heat shock protein (HSP) is progressed, and as a result, immune of a host is activated whereby an effect of hyperthermia of cancer is heightened.

That is, the present invention is an immunostimulator containing a heat shock protein-inducing compound in hyperthermia of cancer.

In the cancers in the present invention, any cancers which can be treated by hyperthermia are included, in particular, malignant brain tumor such as glioblastoma, etc., melanoma (malignant melanoma), breast cancer, prostate cancer, and the like are preferred.

In the hyperthermia of cancer according to the present invention, any therapeutic methods for treating cancer by heating tumor tissue are included, and preferably hyperthermia using magnetic fine particles. The magnetic fine particles mean fine particles having magnetism such as iron, cobalt, nickel, etc. and their compounds, etc., in particular, fine particles having magnetism such as magnetite, etc. A diameter of the magnetic fine particles is 10 to 70 nm, preferably about 40 nm.

The hyperthermia of cancer using the magnetic fine particles is a hyperthermia of cancer using magnetic fine particles as a heat generating material. In this therapeutic method, as a heat generating material, magnetic fine particles that are coated with a cationic phospholipid, or in some cases, CMC-magnetic fine particles in which a cationic liposome (CL) which is a liposome to which an antibody specific to cancer cells is fixed or magnetic fine particles are dispersed in a carboxymethylcellulose (CMC) solution may be used, or else, a needle-shaped molded product in which the magnetic fine particles are molded and solidified into a needle-shape may be used.

The cationic liposome (CL) in the present invention is a material in which magnetic fine particles such as magnetite, etc. are coated by double membranes of phospholipid containing a cationic lipid. As the phospholipid, preferred are glycerophospholipid such as phosphatidylcholine, phosphatidylethanolamine, phosphatidylserine, phosphatidylinositol, phosphatidylglycerol, diphosphatidylglycerol, phosphatidic acid, etc. and sphingolipid such as sphingomyelin, etc., that may be singly or a mixture thereof, in particular, phosphatidylcholine, phosphatidylethanolamine and phosphatidylglycerol are preferred singly or a mixture thereof. As the cationic lipid, there may be mentioned a cationic lipid with a carbon number of 10 to 18 or so, in particular, N-($\alpha$-trimethylammonioacetyl)-didodecyl-O-glutamate is preferred. A mixing ratio of the phospholipid and the cationic lipid is 10:1 to 1:1, preferably 4:1 to 3:1. A thickness of the phospholipid dual membranes is 5 to 40 nm, preferably about 10 nm.

CL may be prepared, for example, as follows. A chloroform solution of the above-mentioned mixture of the lipids is charged in an eggplant shaped flask, and chloroform is evaporated by a rotary evaporator under reduced pressure whereby the lipids mixture is evaporated to dryness inside of the flask in a film state. Next, magnetic fine particles that are previously made in colloidal state are added to the flask with a suitable amount, and the remaining material is subjected to ultrasonic wave treatment. According to this procedure, CL is prepared. A CL in which magnetite was used as the magnetic fine particles is called to as magnetite cationic liposome (MCL).

The CMC-magnetic fine particles can be produced as mentioned below. Magnetic fine particles in an amount of 0.1 to 1.5 g, preferably 0.3 g are charged in a stirring apparatus, and distilled water is added thereto until an amount thereof finally becomes 10 ml. This is warmed in a thermostat chamber at 6°C, and 10 to 30 ml, preferably 20 ml of a 6% CMC solution is added. After stirring the mixture for 30 minutes, ultrasonic wave treatment was applied for 15 minutes to prepare CMC-magnetic fine particles.

The needle-shaped molded product of the magnetic fine particles can be produced as mentioned below. Magnetic fine particles and CMC are dissolved in water in the weight ratio in the range of 4:1 to 15:1, preferably 8:1 and stirred for 90 minutes. This is molded by using an extrusion type molding machine with a diameter of 0.1 to 2.0 mm and a suitable length, preferably in comply with a size of the tumor, particularly preferably molded with a size of 80 mm, and after natural drying for 2 days, they are prepared by drying under hot wind at 80 to 100°C to prepare the product.

Hyperthermia of cancer using MCL is carried out as follows. The thus prepared MCL as mentioned above is administered to tumor tissue by local administration, artery injection, intravenous injection, abdominal injection, etc., then, the MCL is specifically adsorbed by the tumor tissue. When a magnetic field is irradiated, the tumor tissue to which the MCL is adsorbed is superior in absorption of the electromagnetic wave to that of the body tissue, so that the tumor tissue alone is selectively heated. The tumor tissue that is temperature-sensitive is killed by an induced heat.

The hyperthermia by MCL is capable of regressing tumor (metastasis cancer) at the portion to which no MCL is administered. This shows that anti-tumor immune is induced by the hyperthermia using MCL. The immunostimulator of the present invention provides extremely excellent cancer therapeutic effect by more strongly inducing an immune-induction in the hyperthermia of cancer.

The hyperthermia using the CMC-magnetic fine particles can be carried out according to the therapeutic method using the MCL.

The hyperthermia of cancer using a needle-shaped molded product of magnetic fine particles can,be practiced as mentioned below. The needle-shaped molded product of the magnetic fine particles prepared as mentioned above is applied to the tumor tissue by local injection, etc., and a magnetic field is irradiated thereto, the tumor tissue existing a needle-shaped molded product of the magnetic fine particles is superior in absorption of the electromagnetic wave to that of the body tissue, so that the tumor tissue alone is selectively heated. The tumor tissue that is temperature-sensitive is killed by an induced heat. The hyperthermia using the needle-shaped molded product of the magnetic fine particles is also capable of regressing the tumor (metastasis cancer) at the portion to which no needle-shaped molded product is applied. This means that anti-tumor immune is induced by the hyperthermia using the needle-shaped molded product of the magnetic fine particles. The immunostimulator of the present invention provides extremely excellent cancer therapeutic effect by more strongly inducing an immune-induction in the hyperthermia of cancer

The heat shock protein (HSP) in the present invention means a protein in which synthesis thereof is induced by heat shock, and for example, it may include HSP90, HSP70, HSP60, etc., and particularly preferred is HSP70.

The heat shock protein-inducing compound in the present invention means a compound which promotes induction of a heat shock protein, and may include, for example, an isoprenoid such as geranylgeranyl acetone (GGA), retinoic acid, etc.; a lower aliphatic alcohol such as ethanol, etc.; heavy metal ion such as zinc, cadmium, etc.; arsenic; selenium; an anticancer agent such as TNF-$\alpha$ (tissue necrosis factor $\alpha$), cisplatin, 5-FU (5'-fluoro-uracil), adriamycin, etc.; geldanamycin, herbimycinnad, etc.; lidocaine, etc. as a local anesthetic; carbonylcyanid-3-chlorophenylhydrazone, etc. as an uncoupler; lactacystin, etc. as a proteasome inhibitor, and preferred is an isoprenoid, particularly preferred is GGA.

It is uncertain about the mechanism that the immunostimulator of the present invention markedly heighten therapeutic effects of cancer in the hyperthermia, but it can be considered that in the tumor tissue to which said immunostimulator is administered, synthesis of HSP is more promoted when a thermal stress is applied by the hyperthermia. That is, it can be considered that the HSP promoted in synthesis forms a complex with a tumor antigen peptide, this complex is dissolved out from the tumor tissue, and acts as a tumor vaccine, whereby tumor-specific immune can be effectively induced, and as a result, metastasis of cancer can be controlled.

The immunostimulator of the present invention may be any form such as a liquid agent, an ointment, a solid agent, powder material, etc., and in the point of easiness of administration, etc., it is preferably a form of a liquid agent such as a solution, a suspension, an emulsion, etc.

In the immunostimulator of the present invention, depending on the form of the agent, an excipient such as water, starch, lactose, etc.; a stabilizer such as a pH controller, an antioxidant, etc.; a preservative; a dissolution aid; an emulsifier such as a surfactant, etc.; a dispersant such as sucrose, Gum Arabic, sodium citrate, etc.; a colorant; a lubricant; a binder; a disintegrator; a coating agent may be contained.

(Brief description of the drawings)

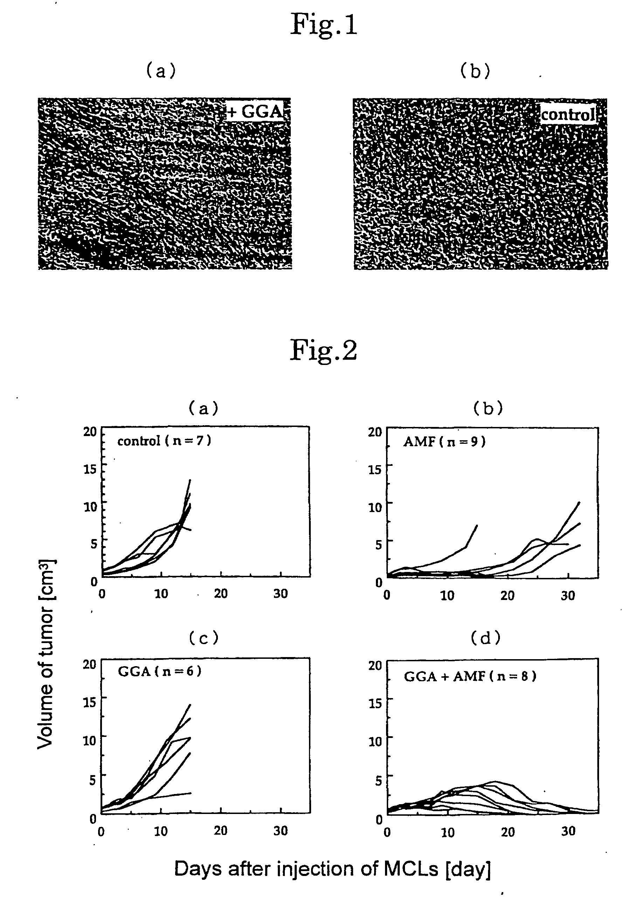

Fig. 1 is a photograph showing a tissue piece of tumor block after MCL administration stained by HSP. (a) is a photograph of the tissue piece when GGA was injected after administration of MCL, and (b) is a photograph of the tissue piece when no GGA was injected after administration of MCL.

Fig. 2 is a drawing showing therapeutic effects of tumor subjected to various kinds of therapy using a volume of the tumor as an index. (a) shows therapeutic effects of the case where MCL alone was administered and no magnetic field irradiation was carried out (Control Group), (b) shows therapeutic effects of the case where MCL alone was administered and magnetic field irradiation was carried out three times in total (AMF Group), (c) shows therapeutic effects of the case where MCL and GGA were administered and no magnetic field irradiation was carried out (GGA Group), and (d) shows therapeutic effects of the case where MCL and GGA are administered and magnetic field irradiation was carried out three times in total ((GGA+AMF) Group) according to the present invention.

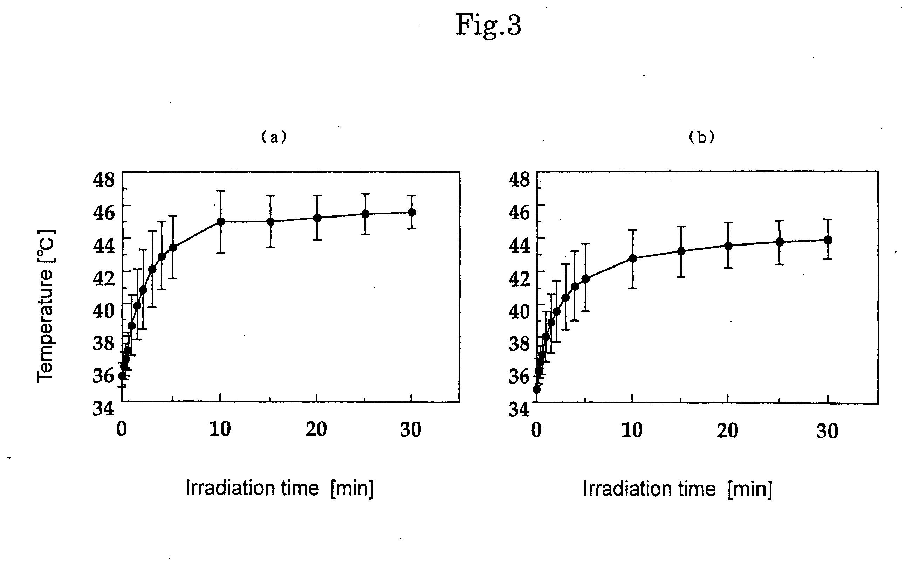

Fig. 3 is a drawing showing a temperature at the tumor surface of rats when the magnetic field irradiation is carried out. (a) shows a temperature at the tumor surface of rats among AMF Group in which tumor was not completely regressed, and (b) shows a temperature at the tumor surface of rats of (GGA+AMF) Group.

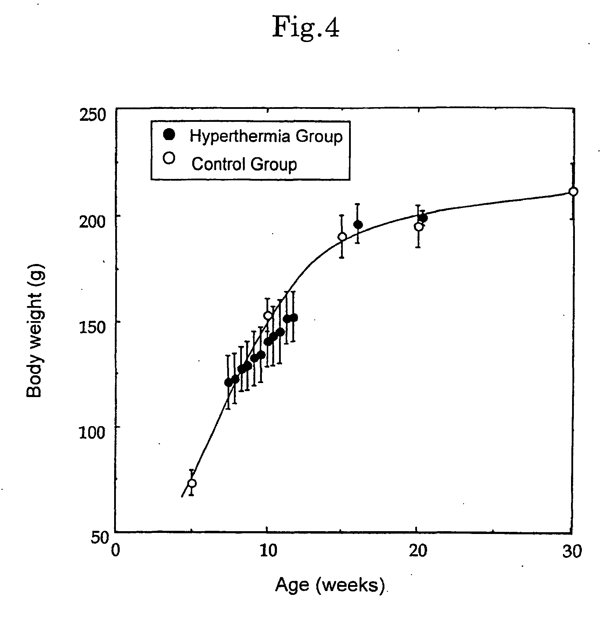

Fig. 4 is a drawing in which change in body weights of rats of the hyperthermia (GGA+AMF) Group in which MCL and GGA are administered and three times of magnetic field irradiation are carried out is compared with those of rats of Control Group in which neither of MCL nor GGA is administered and no magnetic field irradiation was carried out.

(Best mode for carrying out the invention)

An immunostimulator of the present invention is used in combination with hyperthermia of cancer. The immunostimulator of the present invention can be administered to tumor tissue prior to hyperthermia of the tumor tissue, administered to tumor tissue at the time of hyperthermia of the tumor tissue, or else, administered to tumor tissue after hyperthermia of the tumor tissue (preferably within 24 hours after the hyperthermia). In either of the cases, according to administration of the immunostimulator of the present invention, high tumor therapeutic effects can be obtained, and the immunostimulator of the present invention is preferably administered prior to the hyperthermia. Also, the immunostimulator of the present invention may be administered simultaneously with the magnetic fine particles such as MCL, etc., or may be previously administered to the whole body.

An administration method of the immunostimulator of the present invention to tumor tissue may be a local injection, an artery injection, an intravenous injection or an abdominal injection, etc., preferably by a local administration.

An administration dose of the immunostimulator of the present invention may vary depending on symptom, age, sex, etc., of the patient, and for example, it is generally 0.02 mg to 20 mg, preferably 0.1 to 0.5 mg, more preferably about 0.2 mg per 1 g of the tumor tissue.

(Examples)

Example 1 *in vitro* experiment

(A) Cultivation of malignant fibrous histiocytoma (MFH) cells

Cultivation of MFH cells was carried out by using a 100 mm Petri dish for culturing cells into which 10 ml of a medium had been charged, at 37°C for 24 hours in an incubator into which 5% of carbon dioxide was added. As the medium, a medium comprising Dulbecco's modified Eagle's Medium that contains 10% fetal bovine serum and, as antibiotics, penicillin G potassium (100 U/ml) and streptomycin sulfate (90 mg/ml) was used.

(B) Addition of GGA

To the MFH cells cultured for a predetermined time was directly added GGA dissolved in ethanol in a concentration of 0.1M, so that it became a concentration of $10^{-5}$ M or $10^{-4}$ M.

(C) Heating of MFH cells

Immediately after addition of the GGA, a lid of the Petri dish for culturing the MFH cells was closely sealed with a soft plastic film, and the dish was immersed in a thermostat at 45°C for 15 minutes to heat the contents.

(D) Measurement of HSP70

After 6 hours from completion of the heating, a culture broth was removed from the Petri dish for culturing, the cells were washed twice with a physiological saline, a liquid to dissolve the cells attached to a HSP70 immunoassay kit was added to the cells with a predetermined amount to extract HSP70 from the cells. This was quantitated by the immunoassay kit. Also, the cells extract was subjected to electrophoresis, and an amount of HSP70 was qualitatively examined by Western Blot.

(E) Results

As a result of analysis of an expressed amount of the HSP70, the expressed amount of the HSP70 after applying thermal stress was increased depending on an amount of the GGA added.

Example 2 Animal experiment

(A) Experimental individual

As experimental individuals, F344 rats (female, 7 to 8-weeks old) were used. MFH cells $1 \times 10^7$ cultured on Petri dish for cell-cultivation were dispersed in about 50 μl of a physiologically buffered saline, and then, the cells suspension was transplanted subcutaneously to a right leg of an experimental individual by using a scalp vein needle (25Gx5/8") (Terumo Corporation) to make a cancer-carried experimental individual. Nembutal (available from Dainippon Pharmaceutical Co., Ltd.) was used for anesthesia, and that diluted to five times (50 mg/kg body weight) was administered to abdominal cavity.

(B) Preparation of MCL

A slurry of the magnetite (particle diameter 10 nm; available from Toda Kogyo Corporation) was sufficiently washed with a distilled water to remove unnecessary ion components, subjected to ultrasonic wave treatment, to obtain colloidal magnetite. 2 ml of colloidal magnetite (magnetite weight: 40 mg) was added to a phospholipid membrane which had previously been prepared at an inner wall surface of an eggplant shaped flask by using phosphatidylcholine, phosphatidylethanolamine and N-($\alpha$-trimethylammonioacetyl)-didodecyl-o-glutamate with a ratio of 2:2:1 (molar ratio; total lipid amount: 30 mg), and the membrane was swelled by subjecting to vortex stirring. The swelled membrane and the magnetic fine particles were subjected to an ultrasonic wave treatment for 15 minutes (28 W), thereafter 200 μl of 10-fold concentration of a physiological saline was added thereto, and further an ultrasonic wave treatment was carried out for 15 minutes (28W) to obtain MCL.

(C) Administration of MCL

Under anesthesia of a rat, MCL with a concentration of 7.5 mg/ml and 3.3 mg of net weight magnetite was administered to the tumor tissue of the rat 10 days after transplantation of the MFH cells over 30 minutes by using a microsyringe pump (SP100i syringe pump, manufactured by WPI Co.), and thereafter, the rat was allowed to stand for 30 minutes. Magnetic field irradiation was carried out after 24 hours from injection of the MCL.

(D) Preparation and administration of GGA

To 500 mg of Gum Arabic powder was adhered 1 g of GGA, 0.5% of Tween 80 solution was gradually added to the above mixture, to emulsify the mixture under stirring, and a final volume thereof was adjusted to 10 ml. This GGA suspension was diluted with a physiological saline to 100-fold, and 200 μl thereof was locally injected to the tumor tissue by using an injector after 12 hours from administration of MCL.

(E) Magnetic field irradiation

After 24 hours from administration of the MCL, the first magnetic field irradiation was carried out, and thereafter, further magnetic field irradiation was carried out twice with an interval of 24 hours. Accordingly, the magnetic field irradiation was carried out three times in total. The magnetic field irradiation was carried out by using an alternating magnetic field generating device (manufactured by Dai-ichi High Frequency Co., Ltd.). A high frequency magnetic field was a frequency of 120 kHz and a magnetic field strength of 384 Oe, and irradiation was carried out for 30 minutes for each time. A horizontal type coil was used for the magnetic field irradiation, and at this time, care should be taken so that a portion at which the cells were transplanted becomes a center of the coil.

(F) Measurement of temperature at tumor surface

During magnetic field irradiation, a temperature at the tumor surface was measured with a lapse of time. A top end of an optical fiber thermometer was fixed on the surface of the tumor surface with a tape. As a control, a temperature at the rectum was simultaneously measured.

(G) Stain of HSP70

After 2 hours from administration of GGA, the tumor block was cut out, the tissue was fixed with 10% formalin, and after paraffin embedding, a cut piece was cut out, subjected to a paraffin removing treatment, and immuno-stained by using an anti-HSP70 antibody.

(H) Measurement method of tumor tissue volume

Under anesthesia of experimental individuals, a longer diameter and a shorter diameter of the tumor tissue were measured by using calipers and calculated out from the following formula.

$$\text{Tumor tissue volume} = (\text{longer diameter}) \times (\text{shorter diameter})^2 \times 0.5$$

(I) Results

As shown in Fig. 1, as a result of HSP70 stain, in the tumor tissue to which the GGA had been administered, induction of HSP70 was confirmed.

As can be clearly seen from Fig. 2, in the Group of hyperthermia alone using MCL (AMF Group), among 9 individuals, tumors of 4 individuals were completely regressed and cured, and in the Group of hyperthermia using MCL and administration of GGA in combination (GGA+AMF), tumors of 8 individuals among 8 individuals were completely regressed and cured.

As far as the temperature at the tumor surface of rats during hyperthermia in which the cancer was not cured by the hyperthermia (AMF Group) using MCL alone is observed, there is no problem in the hyperthermia itself (see Fig. 3).

Also, as can be seen from Fig. 4, a body weight change in rats of the hyperthermia (GGA+AMF) Group in which MCL and GGA are administered and three times of the magnetic field irradiations were carried out according to the present invention is substantially the same as those of the rats in Control Group in which neither MCL nor GGA was administered and no magnetic field irradiation was carried out, so that it can be understand that a side effect of the present invention is extremely little.

(Utilizability in industry)

When the hyperthermia of cancer was carried out by using the immunostimulator of the present invention, a tumor tissue which is difficultly treated by the hyperthermia alone, and simultaneously metastasis of cancer can be also effectively inhibited.

Moreover, according to the present invention, therapy of cancer can be carried out while markedly controlling side effects such as decrease in a body weight, etc.

**Claims**

1.   An immunostimulator in hyperthermia of cancer which comprises a heat shock protein-inducing compound.

2.   The immunostimulator according to Claim 1, wherein the heat shock protein-inducing compound is selected from the group consisting of isoprenoid, a lower fatty alcohol, a heavy metal ion, arsenic, selenium, an anticancer agent, a local anesthetic, an uncoupler and a proteasome inhibitor.

3.   The immunostimulator according to Claim 2, wherein the isoprenoid is geranylgeranyl acetone.

4.   The immunostimulator according to any one of Claims 1 to 3, wherein the hyperthermia of cancer uses magnetic fine particles or their needle-shaped molded products.

5.   The immunostimulator according to Claim 4, wherein the magnetic fine particles are MCL.

6.   Use of a heat shock protein-inducing compound as an immunostimulator for hyperthermia of cancer.

7.   A tumor treating agent in hyperthermia which comprises the immunostimulator according to any one of Claims 1 to 3 and magnetic fine particles or their needle-shaped molded products.

8.   The tumor treating agent according to Claim 7, wherein the magnetic fine particles are MCL.

# Fig.1

（a） （b）

+ GGA

control

# Fig.2

（a） （b）

control ( n = 7 )

AMF ( n = 9 )

（c） （d）

GGA ( n = 6 )

GGA + AMF ( n = 8 )

Volume of tumor [cm³]

Days after injection of MCLs [day]

Fig.3

# Fig.4

## INTERNATIONAL SEARCH REPORT

| International application No. |
| --- |
| PCT/JP01/09381 |

A. CLASSIFICATION OF SUBJECT MATTER
Int.Cl⁷ A61K45/00, 31/121, 33/26, A61P37/04, 35/00

According to International Patent Classification (IPC) or to both national classification and IPC

B. FIELDS SEARCHED

Minimum documentation searched (classification system followed by classification symbols)
Int.Cl⁷ A61K45/00, 31/121, 33/26, A61P37/04, 35/00

Documentation searched other than minimum documentation to the extent that such documents are included in the fields searched

Electronic data base consulted during the international search (name of data base and, where practicable, search terms used)
CAPLUS(STN), MEDLINE(STN), EMBASE(STN), BIOSIS(STN), JICST(JOIS)

C. DOCUMENTS CONSIDERED TO BE RELEVANT

| Category* | Citation of document, with indication, where appropriate, of the relevant passages | Relevant to claim No. |
| --- | --- | --- |
| X<br>Y | Mitsugu YANASE et al.,<br>Magnetite Cationic Liposomes to Kougan-zai no Heiyou<br>ni yoru Onnetsu Kagaku Ryouhou no Kentou,<br>Kagaku Kougaku Ronbun-shu, 1998, Vol. 24, No. 2,<br>pp. 179-183,<br>the whole document | 7,8<br>1-5 |
| Y | YANASE, Mitsugu et al.,<br>Antitumor immunity induction by intracellular<br>hyperthermia using magnetite cationic liposomes.,<br>Japan J. Cancer Res., 1998, Vol.89, No.7, pp.775-782,<br>especially, Abstract | 1-5 |
| Y | GOTTHARDT, Rainer et al.,<br>The anti-cancer drug cisplatin induces Hsp25 in<br>Ehrlich ascites tumor cells by a mechanism different<br>from transcriptional stimulation influencing<br>predominantly Hsp25 translation.,<br>Int. J. Cancer, 1996, Vol.66, No.6, pp.790-795,<br>especially, Abstract | 1 |

☒ Further documents are listed in the continuation of Box C.  ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>22 January, 2002 (22.01.02) | Date of mailing of the international search report<br>12 February, 2002 (12.02.02) |
| --- | --- |
| Name and mailing address of the ISA/<br>Japanese Patent Office | Authorized officer |
| Facsimile No. | Telephone No. |

Form PCT/ISA/210 (second sheet) (July 1992)

## INTERNATIONAL SEARCH REPORT

| | International application No. |
|---|---|
| | PCT/JP01/09381 |

C (Continuation).   DOCUMENTS CONSIDERED TO BE RELEVANT

| Category* | Citation of document, with indication, where appropriate, of the relevant passages | Relevant to claim No. |
|---|---|---|
| Y | OESTERREICH, Steffi et al., Cisplatin induces the small heatshock protein HSP25 and thermotolerance inEhrlich ascites tumor cells., Biochem. Biophys. Res. Commun., 1991, Vol.180, No.1, pp. 243-8, especially, SUMMARY | 1 |
| Y | TSURUMA, T. et al., Induction of heat shock protein-70 (hsp-70) by intraarterial administration ofgeranylgeranylacetone., Transplant. Proc. 2000, Vol.32, No.7, pp.1631-1633, especially, Abstract | 3 |
| Y | HIRAKAWA, Tetsuya et al., Geranylgeranylacetone induces heatshock proteins in cultured guinea pig gastric mucosal cells and rat gastric mucosa., Gastroenterology, 1996, Vol.111, No.2, pp.345-357, especially, Abstract | 3 |

Form PCT/ISA/210 (continuation of second sheet) (July 1992)

## INTERNATIONAL SEARCH REPORT

| International application No. |
|---|
| PCT/JP01/09381 |

**Box I   Observations where certain claims were found unsearchable (Continuation of item 1 of first sheet)**

This international search report has not been established in respect of certain claims under Article 17(2)(a) for the following reasons:

1. ☒ Claims Nos.: 6
   because they relate to subject matter not required to be searched by this Authority, namely:

   Claim 6 pertains to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box II   Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**   ☐ The additional search fees were accompanied by the applicant's protest.
☐ No protest accompanied the payment of additional search fees.

Form PCT/ISA/210 (continuation of first sheet (1)) (July 1992)